# EUROPEAN PATENT SPECIFICATION

(11) **EP 2 646 031 B1**
(45) Date of publication and mention of the grant of the patent: **22.02.2017**
(21) Application number: 11845186.3
(22) Date of filing: 02.12.2011
(51) Int. Cl.: A61K 31/485, A61K 31/137, A61P 3/04

(54) **INCREASING DRUG BIOAVAILABILITY IN NALTREXONE THERAPY**
ERHÖHUNG DER BIOVERFÜGBARKEIT VON HEILMITTELN IN EINER NALTREXONTHERAPIE
AUGMENTATION DE LA BIODISPONIBILITÉ PHARMACOLOGIQUE D'UN TRAITEMENT À BASE DE NALTREXONE

(30) Priority: 03.12.2010 US 419395 P
(43) Date of publication of application: 09.10.2013
(73) Proprietor: Orexigen Therapeutics, Inc., La Jolla, CA 92037 (US)
(72) Inventor: FLANAGAN, Shawn, San Diego CA 92128 (US); DUNAYEVICH, Eduardo, Westlake Village, CA 91362 (US)
(74) Representative: Zacco Denmark A/S
(86) International application number: PCT/US2011/063177
(87) International publication number: WO 2012/075459

(56) References cited:
- US-A1- 2008 058 407
- US-A1- 2008 110 792
- US-A1- 2008 113 026
- US-A1- 2010 166 889
- US-B2- 7 375 111
- US-B2- 7 682 633
- US-B2- 7 682 633
- GREENWAY FRANK L. ET AL: "Comparison of Combined Bupropion and Naltrexone Therapy for Obesity with Monotherapy and Placebo", JOURNAL OF CLINICAL ENDOCRINOLOGY AND METABOLISM, vol. 94, no. 12, December 2009 (2009-12), pages 4898-4906, XP055100385, US ISSN: 0021-972X, DOI: 10.1210/jc.2009-1350
- HAUSENLOY D J: "Contrave(TM): Novel treatment for obesity", CLINICAL LIPIDOLOGY 2009 FUTURE MEDICINE LTD. GBR, vol. 4, no. 3, June 2009 (2009-06), pages 279-285, XP009176559, ISSN: 1746-0875
- JOHNSON FRANKLIN ET AL: "Food effects on the pharmacokinetics of morphine sulfate and naltrexone hydrochloride extended release capsules", ADVANCES IN THERAPY, vol. 27, no. 11, 14 October 2010 (2010-10-14), pages 846-858, XP055104393, ISSN: 0741-238X, DOI: 10.1007/s12325-010-0074-x
- MIDHA K K ET AL: "Exposure measures applied to the bioequivalence of two sustained release formulations of bupropion.", INTERNATIONAL JOURNAL OF CLINICAL PHARMACOLOGY AND THERAPEUTICS MAY 2005, vol. 43, no. 5, May 2005 (2005-05), pages 244-254, XP009176514, ISSN: 0946-1965
- WADDEN ET AL.: 'Weight Loss with Naltexone Sr/Bupropion Sr Combination Therapy as an Adjunct to Behavior Modification: The COR-BMOD Trial.' OBESITY vol. 19, no. 1, 17 June 2010, pages 110 - 120, XP055100388
- WILCOXA ET AL.: 'An open-label study of naltrexone and bupropion combination therapy for smoking cessation in overweight and obese subjects.' ADDICTIVE BEHAVIORS vol. 35, March 2010, pages 229 - 234, 230, XP026809862
- GREENWAY ET AL.: 'Comparison of Combined Bupropion and Naltrexone Therapy for Obesity with Monotherapy and Placebo.' J CLIN ENDOCRINOL METAB vol. 94, no. 12, 2009, pages 4898 - 4906, XP055100385

## Description

The present application claims priority to U.S. Provisional Patent Application Serial No. 61/419,395, filed on December 3, 2010.

### BACKGROUND OF THE INVENTION

### Field of the Invention

The present invention relates to compositions for use in increasing drug bioavailability in a naltrexone therapy.

### Description of the Related Art

Drug therapies utilizing naltrexone, including in a combination therapy with bupropion, are being investigated for the treatment of a variety of medical conditions, including overweight and obesity, cardiovascular risk factors, insulin resistance, food cravings, visceral fat conditions, smoking, and major depression. Despite the potential use of naltrexone in daily or otherwise regular therapies, currently approved prescribing information for naltrexone-containing products does not refer to studies that examine the effects of food on pharmacokinetics. Further, currently approved prescribing information for WELLBUTRIN SR® characterizes the effects of food on bupropion exposure, but reports only an 11% increase in maximal plasma concentration (Cₘₐₓ) and a 17% increase in area under the concentration time curve (AUC). As a result, there is no guidance or restriction in the prescribing information for naltrexone, alone or in combination with bupropion, with respect to food or food effects. This is illustrated in US 7,375,111 relating to a pharmaceutical composition containing naltrexone and bupropion. A further illustration of the art is seen in Greenway, F.L., et al., J. Clin. Endocrinology and Metabolism, 94(12), 2009, pp. 4898-4906, relating to an immediate release composition of naltrexone in combination with sustained release bupropion in inducing weight loss. A further illustration of the art, Hausenloy, D. J., Clin. Lipidology, 4(3), 2009, pp. 279-285, relates to a fixed-dose combination of sustained release bupropion and sustained release naltrexone for the treatment of obesity.

### SUMMARY OF THE INVENTION

Unexpected food effects have now been identified for drug therapies that utilize naltrexone. Described herein are clinical trials that reveal that the administration of naltrexone and bupropion with food unexpectedly increases the bioavailability of each of these drugs, indicating a positive food effect. For example, the administration of a weight loss treatment comprising naltrexone and bupropion with a high fat, high calorie diet to an overweight or obese individual improves the Cₘₐₓ and AUC of each of these drugs, thereby improving the efficacy of the weight loss treatment. Although one would not ordinarily recommend, administer, or take a high fat, high calorie diet in conjunction with treatments that typically entail dietary restrictions (e.g., treatments for overweight or obesity, cardiovascular risk factors, insulin resistance, food cravings, or visceral fat conditions), the findings described herein enables therapies that involve the administration of naltrexone monotherapy or combined therapies with a wide range of foods.

In view of the observations described herein, a need exists for methods that account for positive food effects associated with naltrexone monotherapy and combined therapies, including methods of increasing drug bioavailability, providing enhanced therapies, and providing information to individuals regarding these effects.

In an embodiment, the invention relates to a pharmaceutical composition for use in the treatment of overweight or obesity comprising a sustained release formulation of naltrexone or a pharmaceutically acceptable salt thereof and a sustained release formulation of bupropion or a pharmaceutically acceptable salt thereof, wherein said composition is administrated in combination with food.

In one aspect the invention relates to a composition for use according to the claims wherein naltrexone or a pharmaceutically acceptable salt thereof is in an amount ranging from about 4 mg to about 32 mg per day with food.

In any embodiment disclosed herein, the amount of the naltrexone or pharmaceutically acceptable salt thereof is in the range of about 4 mg to about 32 mg per day; the amount of the bupropion or pharmaceutically acceptable salt thereof is in the range of about 90 mg to about 360 mg per day; the naltrexone or pharmaceutically acceptable salt thereof comprises a non-sequestered formulation of naltrexone or a pharmaceutically acceptable salt thereof; at least one of the naltrexone or pharmaceutically acceptable salt thereof and the bupropion or pharmaceutically acceptable salt thereof is in a sustained release formulation; each of the naltrexone or pharmaceutically acceptable salt thereof and the bupropion or pharmaceutically acceptable salt thereof is in a sustained release formulation; the naltrexone or pharmaceutically acceptable salt thereof is administered concurrently with the bupropion or pharmaceutically acceptable salt thereof; the naltrexone or pharmaceutically acceptable salt thereof is administered prior to or subsequent to the bupropion or pharmaceutically acceptable salt thereof; the naltrexone or pharmaceutically acceptable salt thereof and the bupropion or pharmaceutically acceptable salt thereof are in a single dosage form; the single dosage form is selected from the group consisting of a pill, a tablet, and a capsule; the naltrexone or pharmaceutically acceptable salt thereof and the bupropion or pharmaceutically acceptable salt thereof are administered or are suitable for administration one or more times per day; the naltrexone or pharmaceutically acceptable salt thereof and the bupropion or pharmaceutically acceptable salt thereof are administered or are suitable for administration two or more times per day; the weight loss activity of the naltrexone or pharmaceutically acceptable salt thereof and the bupropion or pharmaceutically acceptable salt thereof is enhanced compared to the administration of the same amount of either compound alone; the naltrexone or pharmaceutically acceptable salt thereof is in a sustained release formulation; the bupropion or pharmaceutically acceptable salt thereof is in a sustained release formulation; and/or the naltrexone or pharmaceutically acceptable salt thereof and the bupropion or pharmaceutically acceptable salt thereof are together in a single dosage form, where the single dosage form comprises, or comprises about, 4 mg, 8 mg, or 16 mg of the naltrexone or pharmaceutically acceptable salt thereof and comprises, or comprises about, 90 mg or 180 mg of the bupropion or pharmaceutically acceptable salt thereof, and where each of the naltrexone or pharmaceutically acceptable salt thereof and the bupropion or pharmaceutically acceptable salt thereof is in a sustained release formulation.

In any embodiment disclosed herein, the bioavailability of the naltrexone or pharmaceutically acceptable salt thereof is increased compared to the bioavailability of the same amount of the naltrexone or pharmaceutically acceptable salt thereof administered without food; increasing the bioavailability comprises increasing the maximal plasma concentration (Cₘₐₓ) or the extent of absorption (AUC) of the naltrexone or pharmaceutically acceptable salt thereof; the increase in bioavailability comprises an increase in Cₘₐₓ in the range of about 91% to about 271% and an increase in AUC in the range of about 70% to about 107% for the naltrexone or pharmaceutically acceptable salt thereof when taken with a meal compared to the same amount of the naltrexone or pharmaceutically acceptable salt thereof taken during a fasted condition; the increase in bioavailability comprises about a 3.7-fold increase in Cₘₐₓ and about a 2.1-fold increase in AUC for the naltrexone or pharmaceutically acceptable salt thereof when taken with a meal compared to the same amount of the naltrexone or pharmaceutically acceptable salt thereof taken during a fasted condition; and/or the increase in bioavailability comprises about a 1.9-fold increase in Cₘₐₓ and about a 1.7-fold increase in AUC for the naltrexone or pharmaceutically acceptable salt thereof when taken with a meal compared to the same amount of the naltrexone or pharmaceutically acceptable salt thereof taken during a fasted condition.

In any embodiment disclosed herein, the composition for use according to the invention further enables administering the naltrexone or pharmaceutically acceptable salt thereof and the bupropion or pharmaceutically acceptable salt thereof in multiple time-spaced doses, where at least one of the time-spaced doses is administered with food; the method further comprises administering the naltrexone or pharmaceutically acceptable salt thereof and the bupropion or pharmaceutically acceptable salt thereof in multiple time-spaced doses, where each of the time-spaced doses is administered with food; the method further comprises providing or administering bupropion or a pharmaceutically acceptable salt thereof; the naltrexone or pharmaceutically acceptable salt thereof is provided or administered in an amount in the range of, or of about, 4 mg to 32 mg per day; and/or the bupropion or pharmaceutically acceptable salt thereof is provided or administered in an amount in the range of, or of about, 90 mg to 360 mg per day.

In any embodiment disclosed herein, the food comprises a high-fat meal; and/or the food comprises a meal selected from the group consisting of a moderate-calorie, moderate-fat meal of, or of about, 575 calories and fat accounting for, or for about, 23% of the total calorie content, a high-calorie, high-fat meal of, or of about, 1000 calories and fat accounting for, or for about, 50% of the total calorie content, and a meal that falls within a range defined by the moderate-calorie, moderate-fat meal and the high-calorie, high-fat meal.

In any embodiment disclosed herein, the individual is overweight or obese; and/or the individual suffers from obesity or overweight, and a therapeutically effective amount of the naltrexone or pharmaceutically acceptable salt thereof and the bupropion or pharmaceutically acceptable salt thereof is provided or administered to treat the obesity or overweight.

In any embodiment disclosed herein, the naltrexone or a pharmaceutically acceptable salt thereof is administered to the individual in accordance with instructions described herein; the printed information indicates that the increase in bioavailability comprises an increase in the Cₘₐₓ or AUC of the naltrexone or pharmaceutically acceptable salt thereof; the printed information indicates an increase in Cₘₐₓ between, or between about, 91% to 271% and an increase in AUC between, or between about, 70% to 107% for the naltrexone or pharmaceutically acceptable salt thereof when taken with a meal compared to the same amount of the naltrexone or pharmaceutically acceptable salt thereof taken during a fasted condition; the printed information further states that taking the naltrexone or pharmaceutically acceptable salt thereof with food results in an increase in the bioavailability of the naltrexone or pharmaceutically acceptable salt thereof compared to taking the same amount of the naltrexone or pharmaceutically acceptable salt thereof without food;

In any embodiment disclosed herein, the composition for use according to the invention further comprises at least one dosage form comprising bupropion or a pharmaceutically acceptable salt thereof; the composition for use according to the invention enables dosages for multiple days, where the dosage of the naltrexone or pharmaceutically acceptable salt thereof is in the range of, or of about, 4 mg to 32 mg per day; composition for use according to the invention enables dosages for multiple days, where the dosage of the bupropion or pharmaceutically acceptable salt thereof is in the range of, or of about, 90 mg to 360 mg per day; and/or the composition for use further comprises a container, where the container comprises the at least one dosage form, and where the printed information is associated with the container.

In any embodiment disclosed herein, the composition for use for the naltrexone or pharmaceutically acceptable salt thereof and the bupropion or pharmaceutically acceptable salt thereof is, or is about, 8 mg of the naltrexone or a pharmaceutically acceptable salt thereof and is, or is about, 90 mg of the bupropion or a pharmaceutically acceptable salt thereof for the first week of treatment; is, or is about, 16 mg of the naltrexone or a pharmaceutically acceptable salt thereof and is, or is about, 180 mg of the bupropion or a pharmaceutically acceptable salt thereof for the second week of treatment; is, or is about, 24 mg of the naltrexone or a pharmaceutically acceptable salt thereof and is, or is about, 270 mg of the bupropion or a pharmaceutically acceptable salt thereof for the third week of treatment; and is, or is about, 32 mg of the naltrexone or a pharmaceutically acceptable salt thereof and is, or is about, 360 mg of the bupropion or a pharmaceutically acceptable salt thereof for the fourth and any subsequent weeks of treatment.

In any embodiment disclosed herein, the naltrexone or pharmaceutically acceptable salt thereof and the bupropion or pharmaceutically acceptable salt thereof are administered as two 8 mg tablets of sustained-release naltrexone twice daily and two 90 mg tablets of sustained-release bupropion twice daily.

In any embodiment disclosed herein, the naltrexone or pharmaceutically acceptable salt thereof and the bupropion or pharmaceutically acceptable salt thereof are administered for at least 28 weeks; or the naltrexone or pharmaceutically acceptable salt thereof and the bupropion or pharmaceutically acceptable salt thereof are administered for at least 56 weeks.

The invention further enables the use of naltrexone or a pharmaceutically acceptable salt thereof and bupropion or a pharmaceutically acceptable salt thereof in the preparation of a medicament for treating overweight or obesity as described in any of the embodiments disclosed herein.

The invention enables the use of naltrexone or a pharmaceutically acceptable salt thereof and bupropion or a pharmaceutically acceptable salt thereof in the preparation of a medicament for increasing drug bioavailability in combined naltrexone/bupropion weight loss therapy as described in any of the embodiments disclosed herein.

The invention enables the use of naltrexone or a pharmaceutically acceptable salt thereof and bupropion or a pharmaceutically acceptable salt thereof in the preparation of a medicament for providing enhanced naltrexone therapy to an individual as described in any of the embodiments disclosed herein.

The invention enables the use of naltrexone or a pharmaceutically acceptable salt thereof and bupropion or a pharmaceutically acceptable salt thereof in the preparation of a medicament for providing a weight loss regimen to an individual as described in any of the embodiments disclosed herein.

The invention enables the use of naltrexone or a pharmaceutically acceptable salt thereof and bupropion or a pharmaceutically acceptable salt thereof in the preparation of a medicament for maximizing the efficacy of a treatment for overweight or obesity as described in any of the embodiments disclosed herein.

The invention enables the use of naltrexone or a pharmaceutically acceptable salt thereof and bupropion or a pharmaceutically acceptable salt thereof in the preparation of a medicament for improving patient compliance with instructions to take a weight loss composition with food as described in any of the embodiments disclosed herein.

The invention enables the use of naltrexone or a pharmaceutically acceptable salt thereof and bupropion or a pharmaceutically acceptable salt thereof in the preparation of a medicament for providing an FDA-approved weight loss drug as described in any of the embodiments disclosed herein.

### DETAILED DESCRIPTION OF THE PREFERRED EMBODIMENT

Various embodiments described herein enables methods of increasing the bioavailability of naltrexone and/or bupropion in naltrexone and combined naltrexone/bupropion therapies. Increasing the bioavailability of naltrexone and/or bupropion can improve a variety of therapies. For example, increased bioavailability can result in more effective dosing. In some embodiments, more effective dosing allows for a lower dosage of naltrexone and/or bupropion to be administered to an individual. In some embodiments, administration of naltrexone and/or bupropion with food can also reduce the frequency and/or severity of adverse effects associated with naltrexone, bupropion, or other drugs. In some embodiments, a reduction in side effects results in improved patient compliance with a treatment. The administration of naltrexone or combined naltrexone/bupropion therapies with food can also generally improve the consistency of pharmacokinetics associated with naltrexone or combined naltrexone/bupropion therapies as variability tends to be highest when bioavailability is low. In some embodiments, this improved consistency allows for greater dosing certainty and improved safety and/or tolerability for naltrexone, bupropion, or other drugs.

The term "bupropion" may be used in a general way herein to refer to a free base of bupropion, a pharmaceutically acceptable bupropion salt (including anhydrous forms, e.g., anhydrous bupropion), a bupropion metabolite (e.g., hydroxybupropion, threohydrobupropion, and erythrohydrobupropion), a bupropion isomer, or mixtures thereof. Reference herein to "bupropion" will be understood as encompassing all such forms, unless the context clearly indicates otherwise.

The term "naltrexone" may be used in a general way herein to refer to a free base of naltrexone, a pharmaceutically acceptable naltrexone salt (including hydrates and anhydrous forms, e.g., naltrexone hydrochloride dihydrate and anhydrous naltrexone hydrochloride), a naltrexone metabolite, a naltrexone isomer, or mixtures thereof. Reference herein to "naltrexone" will be understood as encompassing all such forms, unless the context clearly indicates otherwise.

The term "pharmaceutically acceptable salt," as used herein, refers to a formulation of a compound that does not cause significant irritation to an organism to which it is administered and does not abrogate the biological activity and properties of the compound. Pharmaceutical salts can be obtained by routine experimentation. Non-limiting examples of pharmaceutically acceptable salts include bupropion hydrochloride, radafaxine hydrochloride, naltrexone hydrochloride, and 6-β naltrexol hydrochloride.

Throughout the present disclosure, when a particular compound is mentioned by name, for example, bupropion or naltrexone, it is understood that the scope of the present disclosure encompasses pharmaceutically acceptable salts, esters, amides, or metabolites of the named compound. For example, in any of the embodiments herein, an active metabolite of naltrexone (e.g., 6-β naltrexol) can be used in combination with, or instead of, naltrexone. In any of the embodiments herein, an active metabolite of bupropion, including S,S-hydroxybupropion (i.e., radafaxine), can be used in combination with, or instead of, bupropion.

The term "sustained release," as used herein, has its ordinary meaning as understood by those skilled in the art and thus includes, by way of non-limiting example, the controlled release of a drug from a dosage form over an extended period of time. For example, in some embodiments, sustained-release dosage forms are those that have a release rate that is slower that of a comparable immediate release form, e.g., less than 80% of the release rate of an immediate-release dosage form.

Those skilled in the art will understand that an immediate-release naltrexone formulation appropriate for use as a reference standard is the immediate-release naltrexone formulation, widely available commercially as the REVIA® brand of naltrexone hydrochloride, or an equivalent thereof. Those skilled in the art will also understand that an immediate-release bupropion formulation appropriate for use as a reference standard is the immediate-release bupropion formulation, widely available commercially as the WELLBUTRIN® brand of bupropion, or an equivalent thereof. The U.S. government regulates the manner in which prescription drugs can be labeled and thus reference herein to the REVIA® brand of naltrexone hydrochloride and WELLBUTRIN® brand of bupropion have well-known, fixed, and definite meanings to those skilled in the art.

The term "oral dosage form," as used herein, has its ordinary meaning as understood by those skilled in the art and thus includes, by way of non-limiting example, a formulation of a drug or drugs in a form administrable to a human, including pills, tablets, cores, capsules, caplets, loose powder, solutions, and suspensions.

The term "food effect," as used herein, refers to a phenomenon that can influence the absorption of drugs following administration. A food effect can be designated "negative" when absorption is decreased, or "positive" when absorption is increased and manifested as an increase in bioavailability (e.g., as reflected by AUC). Food effects can also refer to changes in maximum concentration (Cₘₐₓ), or the time to reach maximum concentration (Tₘₐₓ), independently of overall absorption. As a result, some drugs can preferably be taken in either fasted or fed conditions to achieve an optimum desired effect. As used herein, the terms "with food" and "fed" can be used interchangeably. As used herein, the terms "without food," "fasted," and "fasting" can be used interchangeably.

The terms "mitigate" or "mitigation" of weight gain, as used herein, include preventing or decreasing the amount of weight gain associated, e.g., with the administration of a drug or a change in life activity. In some embodiments, mitigation of weight gain is measured relative to the amount of weight gain typically experienced when only one or neither of naltrexone or bupropion is administered.

The term "promotion" of weight loss, as used herein, includes causing weight loss relative to a baseline weight for a least a portion of the period of treatment. This includes an individual that initially gains some weight, but during the course of treatment loses weight relative to a baseline prior to beginning treatment, as well as individuals that regain a portion or all of the weight that is lost by the end of the treatment period. In a preferred embodiment, at the end of the treatment period, the individual has lost weight relative to a baseline. In a preferred embodiment, mitigation of weight gain or promotion of weight loss in a patient administered naltrexone and bupropion is greater than when neither or only one of naltrexone or bupropion is administered, and more preferably an at least additive, or better than additive, or synergistic, effect of administering the two compounds is achieved.

The terms "pharmacokinetic profile" or "pharmacokinetics," as used herein, have their ordinary meaning as understood by those skilled in the art and thus include, by way of non-limiting example, a characteristic of the curve that results from plotting concentration (e.g. blood plasma or serum) of a drug over time, following administration of the drug to a subject. A pharmacokinetic profile thus includes a pharmacokinetic parameter or set of parameters that can be used to characterize the pharmacokinetics of a particular drug or dosage form when administered to a suitable population. Various pharmacokinetic parameters are known to those skilled in the art, including area under the concentration vs. time curve (AUC), area under the concentration time curve from time zero until last quantifiable sample time (AUCo-ₜ), area under the concentration time curve from time zero extrapolated to infinity (AUCo-oo), area under the concentration time curve over the steady state dosing interval or from time zero to twelve hours (AUCo-₁₂) for twice-daily dosing, maximum concentration (e.g. blood plasma/serum) after administration (Cₘₐₓ), and time to reach maximum concentration (e.g. blood plasma/serum) after administration (Tₘₐₓ). AUCiₐₛₜ indicates the area under the blood plasma concentration vs. time curve from the time of administration until the time of the last time point. Pharmacokinetic parameters may be measured in various ways known to those skilled in the art, e.g., for single dosage or steady-state. Differences in one or more of the pharmacokinetic profiles (e.g., Cₘₐₓ) may indicate pharmacokinetic distinctness between two formulations.

The term "sequestered," as used herein, refers to a drug that is not substantially released following the administration of a dosage form comprising the drug. For example, dosage forms comprising morphine sulfate and naltrexone have been formulated in a manner that greatly reduces in vivo release of naltrexone following the administration of the intact version of the dosage form, e.g., as described in U.S. Patent Publication No. 2009/0162450.

Those skilled in the art will understand that pharmacokinetic parameters may be determined by comparison to a reference standard using clinical trial methods known and accepted by those skilled in the art, e.g., as described in the examples set forth herein. Since the pharmacokinetics of a drug can vary from patient to patient, such clinical trials generally involve multiple patients and appropriate statistical analyses of the resulting data {e.g., ANOVA at 90% confidence). Comparisons of pharmacokinetic parameters can be on a dose-adjusted basis, as understood by those skilled in the art.

Pharmacokinetic profiles can be determined by analyzing a patient population that has received a treatment of naltrexone alone, or a combined treatment of naltrexone and one or more other drugs (such as bupropion), with food, and comparing them to a comparable patient population that has received the same treatment without food. In some embodiments, the pharmacokinetic properties are measured after a single-drug dosage, while in others, they are measured at steady-state. In some embodiments, pharmacokinetics can be determined by monitoring a plasma naltrexone and/or active naltrexone metabolite (e.g., 6β-naltrexol) concentration profile. In some embodiments, pharmacokinetics can be determined by monitoring a plasma bupropion and/or active bupropion metabolite (e.g., threohydrobupropion) concentration profile.

An increase in bioavailability can be determined using one or more measures known to one of skill in the art, such as an increase in AUC, Cₘₐₓ, or Tₘₐₓ, which can each independently be an increase that is, is about, is at least, is at least about, 5%, 10%, 20%, 30%, 40%, 50%, 75%, 100%, 125%, 150%, 175%, 200%, 250%, 300%, 350%, 400%, 450%, 500%, 600%, 700%, or more, or within a range defined by any two of these values (e.g., 5%-500%, 10%-400%, or 20%-300%), wherein the increase is as compared to a reference treatment (e.g., a fasted state or a different fed state).

In preferred embodiments, the bioavailability of naltrexone, bupropion, or metabolites thereof falls outside the standard 80% - 125% range for bioequivalence. For example, the 90% confidence interval ("CI") for the Cₘₐₓ or AUC for naltrexone may be higher than 125% of the reference treatment (e.g., a fasted state or a different fed state).

In some embodiments, the individual has a body mass index (BMI) of at least 25 kg/m². In some embodiments, the individual has a BMI of at least 30 kg/m². In some embodiments, the individual has a BMI of at least 40 kg/m². In some embodiments, the individual has a BMI of less than 25 kg/m², or develops a BMI less than 25 kg/m² during the course of administration of naltrexone and bupropion. In these embodiments, it may be beneficial for health or cosmetic purposes to mitigate subsequent weight gain or to promote weight loss, thereby reducing the BMI even further. In some embodiments, the individual has been diagnosed by a physician as being overweight or obese. In some embodiments, the individual is identified, including self-identified, as overweight or obese, or is identified as having been diagnosed as overweight or obese.

In some embodiments, the promotion of weight loss is measured by a percent change from a baseline body weight. In some of these embodiments, the amount of weight loss is, is about, is at least, is at least about 0.5%, 1%, 1.5%, 2%, 2.5%, 3%, 4%, 5%, 6%, 7%, 8%, 9%, 10%, 12%, 15%, or more of initial body weight, or a range defined by any two of the preceding values. In some embodiments, the promotion of weight loss is measured as a reduction in weight gain relative to the amount of weight gain experienced when neither or only one of naltrexone and bupropion is administered, and the amount of reduction in weight gain is, is about, is at least, is at least about, 2%, 5%, 10%, 15%, 20%, 25%, 30%, 40%, 50%, 60%, 70%, 80%, 90%, 100%, 105%, 110%, 115%, 120%), or more, or a range defined by any two of the preceding values.

In some embodiments, the mitigation of weight gain is measured by a percent change from a baseline body weight. In some of these embodiments, the amount of weight gain is, is about, is not more than, is not more than about 0%, 0.5%, 1%, 1.5%, 2%, 2.5%, 3%, 4%, 5%, 6%, 7%, 8%, 9%, 10%, or more of initial body weight, or a range defined by any two of the preceding values.

In some embodiments, the dosage of naltrexone and/or bupropion is adjusted so that the patient loses weight at a rate of about 3% of baseline body weight every six months. However, the rate of weight loss for a patient may be adjusted by the treating physician based on the patient's particular needs.

In some embodiments, the mitigation of weight gain or promotion of weight loss occurs by increasing satiety in the individual. In some embodiments, the mitigation of weight gain or promotion of weight loss occurs by suppressing the appetite of the individual. In some embodiments, the treatment comprises instituting a regimen of diet and/or increased activity.

In some embodiments, the naltrexone or combination therapy, including naltrexone in combination with bupropion, is in an amount sufficient to affect weight loss, reduce a cardiovascular risk factor, increase insulin sensitivity, reduce food cravings, treat a visceral fat condition, mitigate weight gain or promote weight loss during smoking cessation, or provide weight loss therapy in patients with major depression. Examples of such methods of treatment are disclosed in U.S. Patent Nos. 7,375, 111 and 7,462,626; in U.S. Patent Publication Nos. 2007/0275970, 2007/0270450, 2007/0117827, 2007/0179168, 2008/0214592, 2007/0128298, and 2007/0129283; in U.S. Patent Application Nos. 12/751970, 61/167486, and 61/293844; and in WO 2009/158114, describing methods of affecting weight loss, reducing cardiovascular risk factors, increasing insulin sensitivity, reducing food cravings, treating visceral fat conditions, mitigating weight gain or promoting weight loss during smoking cessation, and providing weight loss therapy in patients with major depression. In some embodiments, the cardiovascular risk factor includes one or more of the following: total cholesterol level, LDL cholesterol level, HDL cholesterol level, triglyceride level, glucose level, and insulin level. In some embodiments, the cardiovascular risk factor includes one or more of the following: total cholesterol level, HDL cholesterol level, and triglyceride level.

In some embodiments, the increased efficacy of a weight loss treatment described herein comprises an improvement in an outcome measure. For example, in some embodiments, the increased efficacy increases the amount of weight loss. In some embodiments, the increase in efficacy decreases the frequency or severity of adverse events, including but not limited to nausea, constipation, vomiting, dizziness, dry mouth, headache, and insomnia. In some embodiments, the increased efficacy improves another secondary endpoint, including but not limited to waist circumference, high- sensitivity C-reactive protein (hs-CRP) levels, triglyceride levels, HDL cholesterol levels or the ratio of LDL/HDL cholesterol levels. As one of skill in the art will recognize, in some circumstances, it is desirable to decrease waist circumference, hs-CRP levels, triglyceride levels, and the ratio of LDL/HDL cholesterol levels, and to increase HDL cholesterol levels. In some embodiments, the improvement in the outcome measure is, is about, is at least, or is at least about 1, 2, 3, 4, 5, 7, 10, 12, 15, 20 30, 40, 50, 60, 70, 80, 90, or 100%, or within a range defined by any two of these values.

In some embodiments, the amount of weight loss when a composition for use according to the invention is taken with a meal is significantly more than the amount of weight loss when the treatment is taken without food. In some embodiments, the amount of weight loss when the treatment is taken with a high-fat meal is significantly more than the amount of weight loss when the treatment is taken without food. In some embodiments, the amount of weight loss when the treatment is taken with a high-fat meal during a steady state of a combined naltrexone/bupropion therapy is significantly more than the amount of weight loss when the treatment is taken under the same conditions without food.

The exact formulation, route of administration, and dosage for naltrexone and naltrexone combination therapies as described herein can be chosen by the individual physician in view of the patient's condition. {See e.g., Fingl et al. 1975, in "The Pharmacological Basis of Therapeutics," Ch. 1 p. 1).

In some embodiments, naltrexone and bupropion are each divided into equal doses and administered more than once per day. In some embodiments, naltrexone and bupropion are each divided into unequal doses and administered more than once per day. In some embodiments, naltrexone and bupropion are divided into a different number of doses and are administered a different number of times per day. In one such embodiment, the dose of one of naltrexone or bupropion is divided, while the dose of the other is not.

In some embodiments, one or both of naltrexone and bupropion is administered one, two, three, four, or more times per day. Either or both compounds can be administered less than once per day, for example once every 2, 3, 4, 5, 6, 7, 8, 9, 10, 11, 12, 13, or 14 days, or every 1 or 2 weeks, or a range defined by any two of the preceding values. In some embodiments, the number of administrations per day is constant (e.g., one time per day). In other embodiments, the number of administrations is variable. The number of administrations may change depending on effectiveness of the dosage form, observed side effects, external factors {e.g., a change in another medication), or the length of time that the dosage form has been administered.

In some embodiments, the daily dose of naltrexone can range from about 4 mg to about 32 mg, or about 8 mg to about 32 mg, or about 8 mg to about 16 mg. In some embodiments, the daily dose is about 4 mg, about 8 mg, about 12 mg, about 16 mg, about 32 mg, of naltrexone, or a range defined by any two of the preceding values. The selection of a particular dosage may be based on the weight of the patient. The selection of a particular dosage may be based on the identity, dosage, and/or dosing schedule of another co-administered compound. However, in some embodiments, it may be necessary to use dosages outside these ranges. In some embodiments, the daily dose is administered in a single oral dosage form. In some embodiments, the daily dose of naltrexone is the same, and in some embodiments, the daily dose is different.

In some embodiments, the daily dose of bupropion can range from about 90 mg to about 360 mg. In some embodiments, the daily dose is about 90 mg, about 180 mg, about 360 mg of bupropion, or a range defined by any two of the preceding values. The selection of a particular dosage may be based on the weight of the patient. The selection of a particular dosage may be based on the identity, dosage and/or dosing schedule of another co-administered compound. However, in some embodiments, it may be necessary to use dosages outside these ranges. In some embodiments, the daily dose is administered in a single oral dosage form. In some embodiments, the daily dose of bupropion is the same, and in some embodiments, the daily dose is different.

The compositions for use as described herein may be distributed, provided to a patient for self-administration, or administered to an individual. In some embodiments, the combined naltrexone/bupropion therapies include a third compound.

In some embodiments, naltrexone and/or bupropion are provided or administered as an oral dosage form. In some embodiments, the oral dosage form is in the form of a pill, tablet, core, capsule, caplet, loose powder, solution, or suspension. In a preferred embodiment, the oral dosage form is in the form of a pill, tablet, or capsule. In some embodiments, the combined naltrexone/bupropion therapy is provided in a single oral dosage form. In some embodiments, the oral dosage form is in the form of a trilayer tablet as described in U.S. Patent Publication No. 2008/0113026.

In some embodiments, at least one of naltrexone and bupropion is administered with varying frequency during treatment. In some of these embodiments, the varying frequency comprises a decreased frequency over time. For example, one or both of naltrexone and bupropion can be initially administered more than once per day, followed by administration only once per day at a later point in treatment. In some embodiments, the daily dosage of at least one of naltrexone and bupropion is consistent despite the varying frequency of administration. For example, in some embodiments, two tablets of each of naltrexone and bupropion are initially administered twice per day, while four tablets of each of naltrexone and bupropion are administered once per day at a later point in treatment. Alternatively, in some embodiments, one or two tablets of each of naltrexone and bupropion are administered at a later point in treatment, where the one or two tablets have an equivalent total daily dosage as the two tablets each of naltrexone and bupropion initially administered twice per day.

In some embodiments where one or both of naltrexone and bupropion are administered less than once per day in a controlled release or sustained release (SR) formulation, the dose is selected so that the patient receives a daily dose that is about the same as a daily dose described herein.

In some embodiments, the naltrexone, alone or in a combination treatment, is not a sequestered form of naltrexone. For example, in some embodiments, naltrexone is in a non-sequestered, controlled release formulation. In some embodiments, naltrexone is a non-sequestered, sustained release formulation. In preferred embodiments, at least 50% of the naltrexone is released within 24 hours of administration.

In some embodiments, at least one of naltrexone or bupropion is administered in consistent daily dosages throughout the period of treatment. In some embodiments, at least one of naltrexone or bupropion is administered in varying daily dosages during the period of treatment. In some of these embodiments, the daily dosages comprise increasing daily dosages over time. In some of these embodiments, the daily dosages comprise decreasing daily dosages over time.

In some embodiments, naltrexone and bupropion are administered individually. In some embodiments, naltrexone and bupropion are administered in a single pharmaceutical composition comprising naltrexone and bupropion. In some embodiments, at least one of naltrexone or bupropion is in a sustained release or controlled release formulation. For example, sustained release forms of naltrexone are described in U.S. Patent Publication No. 2007/0281021. In some embodiments, at least one of naltrexone or bupropion is administered with a physiologically acceptable carrier, diluent, or excipient, or a combination thereof. Examples of naltrexone/bupropion combinations, formulations thereof, and methods of administering them are disclosed in U.S. Patent Nos. 7,375, 111 and 7,462,626. Techniques for formulation and administration of the compounds of the instant application may be found in "Remington's Pharmaceutical Sciences," Mack Publishing Co., Easton, PA, 18th edition, 1990.

In some embodiments, naltrexone is administered prior to bupropion. In some embodiments, naltrexone is administered subsequent to bupropion. In some embodiments, naltrexone and bupropion are co-administered. As used herein, coadministration includes administration in a single dosage form, or separate dosage forms that are administered at, or nearly at, the same time.

In some embodiments, the administration of naltrexone and bupropion is continued for a period of, or of about, 1, 2, 3, 4, 6, 8, 10, 12, 16, 20, 24, 36, 48, or 52 weeks, or a range defined by any two of the preceding values. In some embodiments, the administration of naltrexone and bupropion is continued until the reduction in symptoms of a disease, disorder, or condition is stabilized for a period of, or of about, 1, 2, 3, 4, 5, 6, or more weeks, or a range defined by any two of the preceding values. For example, in some embodiments, the administration of a combined naltrexone/bupropion therapy is continued until the mitigation of weight gain or promotion of weight loss in an individual is stabilized for a period of, or of about, 1, 2, 3, 4, 5, 6, or more weeks, or a range defined by any two of the preceding values. In some embodiments, administration of naltrexone, or naltrexone and bupropion, is continued until the individual no longer needs a treatment.

### EXAMPLES

The examples below illustrate various aspects of the invention.

### Example 1 : Single Dose Naltrexone and Bupropion

A phase I, open label, randomized, single-dose, three-way crossover study was performed to assess the effects of food on the plasma pharmacokinetics of sustained release naltrexone ("naltrexone SR") / sustained release bupropion ("bupropion SR") combination trilayer tablets. Healthy adult volunteers (n=18; 15 males, 3 females; mean age = 37 y.o.; range = 21-59 y.o.) were randomized to receive each of three treatments under in a crossover design with a minimum 14-day washout between treatments. The treatments consisted of a single dose of either: (1) two naltrexone SR 8 mg / bupropion SR 90 mg tablets (i.e., two "NB 8/90 tablets") under fasted conditions; (2) two NB 8/90 tablets administered shortly after a standardized high-fat meal; or (3) two NB 8/90 tablets administered shortly after a standardized moderate-fat meal. Blood samples for determination of plasma concentrations for naltrexone, bupropion, and their respective metabolites were measured within 15 minutes predose (baseline), and at time points from 0.5-120 hours postdose.

A summary of food effect comparisons in subjects administered NB 8/90 tablets is also provided in Table 1. Table 2 presents the results of statistical comparisons between the fed (i.e., treatment 3) and fasted (i.e., treatment 1) conditions. Administration of the NB 8/90 tablets under high-fat conditions increased naltrexone Cₘₐₓ and AUC values by 271% and 107%, respectively (i.e., 3.7-fold and 2.1-fold the value of the fasted condition, respectively), and increased bupropion Cₘₐₓ and AUC by 80% and 38%, respectively (i.e., 1.8-fold and 1.4-fold the value of the fasted condition, respectively), than those observed with the same tablets administered under fasted conditions. The 90% CIs of the naltrexone and bupropion Cₘₐₓ and AUC percent geometric mean ratios did not fall within the 80% to 125% range. Median Tₘₐₓ values and apparent terminal elimination tm for naltrexone were similar for naltrexone between fed and fasted conditions. For bupropion, median Tₘₐₓ was one hour shorter under fed conditions than fasted conditions; apparent terminal elimination tm values were similar at 22.70 and 20.44 hr, respectively. This indicates that food does not decrease clearance, but rather increases absorption and/or decreases first pass effect.

With respect to metabolites, Cₘₐₓ was increased 52% for óβ-naltrexol, 26%) for threohydrobupropion, and 40% for a pharmacologically weighted composite of bupropion metabolites (PAWC, a single total concentration for all bupropion related active metabolites adjusted for relative potency) under high-fat conditions compared to fasted conditions, and the upper bounds of the 90% CIs for the Cₘₐₓ percent geometric mean ratios of these metabolites were generally above 125%. AUC for all metabolites and PAWC, and Cₘₐₓ for hydroxybupropion and erythrohydrobupropion were comparable between the high-fat and fasted conditions, with all 90% CIs within 80% to 125% range.

Administration of the NB 8/90 tablets in conjunction with a high-fat standardized meal resulted in a positive food effect, with naltrexone Cₘₐₓ and AUC increasing approximately 300%) and 100%), respectively, and bupropion Cₘₐₓ and AUC increasing approximately 80% and 40%, respectively. In addition, administration of the combination with food reduced the adverse events, especially gastrointestinal events such as nausea. Three of 18 patients in the fasted group experienced a drug-related adverse event (17%), while only one of 16 patients in the fed group experienced an adverse event (6%).

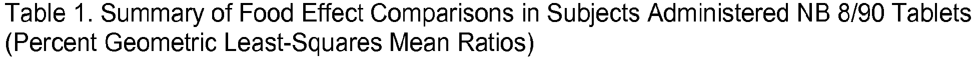

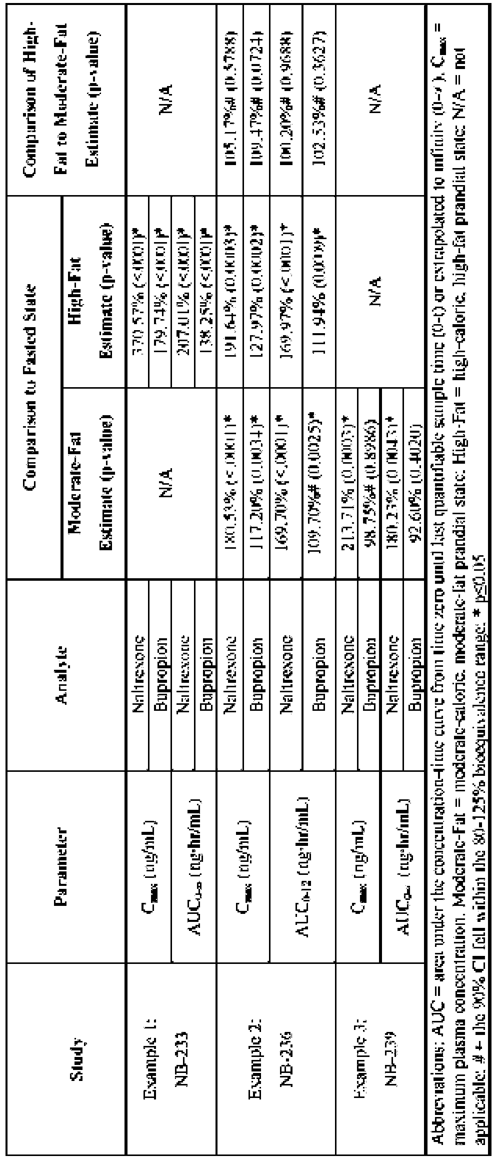

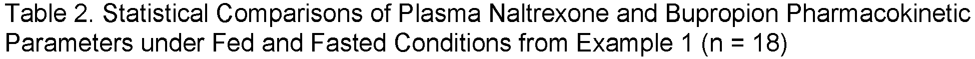

### Example 2: Food Effect on Steady-State Naltrexone and Bupropion

A phase I, open label, steady-state study was performed to assess the plasma pharmacokinetics of NB tablets under fed and fasted conditions. An extension of the study allowed for the evaluation of the effect of food on the pharmacokinetics of NB tablets. Dosing to steady state affords an opportunity to observe accumulation of bupropion and metabolites, and thus better estimate the magnitude of pharmacokinetic interactions expected after chronic administration.

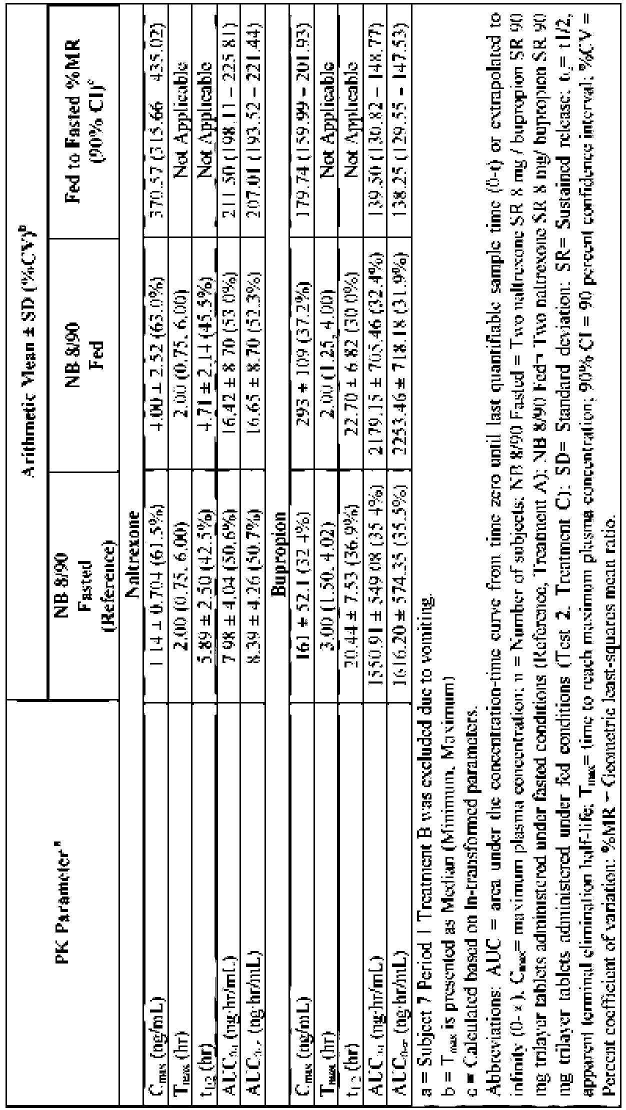

Days 1-31 of the study were devoted to the primary investigation of metoprolol pharmacokinetics in the context of steady-state dosing with NB tablets. Subjects (n=18) received a metoprolol 50 mg IR tablet on Days 1 and 31. NB was dose escalated every week from a single NB 8/90 tablet per day on Day 3 to a maximum dose of NB 32/360 (two NB 8/90 tablets BID) starting on Day 24. In the clinic setting (Days 1-3 and 27-31), standardized meals were provided before dosing and pharmacokinetic sampling. These meals were moderate in fat and caloric content. Subjects were instructed to take study medication with food during the outpatient (Days 4-26) phase of the study.

After Day 31 , subjects (n=I I ; 7 males, 4 females; mean age = 34 y.o.; range = 19-45 y.o.) were given the option to participate in a 3-day treatment extension period designed to assess the effects of food on naltrexone and bupropion pharmacokinetics. The evaluation was designed to enable comparisons between fed conditions (either a "moderate" meal, or a high-fat, high-calorie meal) and between both fed conditions and the fasted state. The treatment sequence of the extension study was as follows:
Morning: Half of Subjects Fasted
Morning; Moderate Meal Half of Subjects High-Fat Meal Morning : Opposite Condition Io Day Evening: Moderate Meal 33 Mommy
Evening; Moderate Meal
Day 32 Day 33 Dov 34

Naltrexone and bupropion pharmacokinetic data following a moderate meal was obtained on Day 30. Subjects enrolling in the optional extension continued BID dosing administration (with moderate meals) through Day 32. On Day 33, subjects were randomized in an approximate 1 : 1 ratio to receive the morning dose of study medication in either the fasted state or in the fed state with a high-fat (57%), high-calorie (910 kcal) meal. The evening dose on Day 33, study medication was given with food ("moderate" meal). For Day 34, subjects received their morning dose under the opposite condition from what they received on the morning of Day 33. On Days 33 and 34, pharmacokinetic samples were collected for naltrexone, bupropion, and their respective metabolites prior to dosing and through 12 hours of sampling following the morning dose. For purposes of studying the effects of food, the comparisons were between Day 30 pharmacokinetics ("moderate" meal) and the fasted and high-fat meal conditions administered on Day 33 and Day 34.

A summary of food effect comparisons in subjects administered NB 8/90 tablets is also provided in Table 1. Table 3 shows a summary and statistical comparison of naltrexone and bupropion steady state pharmacokinetic parameters under different meal conditions. Changing the meal composition prior to the morning dose from moderate-fat, moderate-calorie meal to high-fat, high-calorie meal had no effect on naltrexone Cmaₓ and AUCo-12 parameter values, with the geometric mean ratios and CIs for the comparison contained within 80-125% bio equivalence range. However, the lower boundary of the 90% CI for the comparison of Cmiₙ was below the 80% lower limit. The results for óβ-naltrexol were similar to those for naltrexone, with the exception that the 90% CIs for Cmiₙ fell within the 80-125% range.

Administration of two NB 8/90 tablets following the moderate- fat, moderate-calorie meal and following the high-fat, high-calorie meal resulted in, respectively, 81% and 92%, higher naltrexone Cₘₐₓ values (i.e., 1.8-fold and 1.9-fold the value of the fasted condition) and 70% higher (for both meals) AUCo-12 values (i.e., 1.7-fold the value of the fasted condition) relative to administration under fasting conditions. The effect on Cmiₙ values was less and not consistent, with a 10% higher Cmiₙ for the moderate-fat, moderate-calorie meal versus the fasted condition and a 12% lower Cmiₙ for the high- fat, high-calorie meal versus the fasted condition. In contrast to the effects observed with naltrexone, neither meal type appeared to affect óβ-naltrexol exposure parameters. With the exception of Cmax in the high-fat versus fasted conditions, which was slightly increased, all parameters and 90% CIs fell within the 80-125% range.

Changing the meal composition prior to the morning dose from a moderate-fat, moderate-calorie meal to a high-fat, high-calorie meal had no effect on bupropion absorption from two NB 8/90 tablets. The geometric mean ratios and 90% CIs for the comparisons of bupropion Cmax, Cmin, and AUCo-12 parameters following high-fat, high-calorie versus moderate- fat, moderate-calorie meal indicated a change of -10% in Cmax, and all parameters were entirely contained within the 80-125% bioequivalence range.

Administration of two NB 8/90 tablets following a moderate- fat, moderate-calorie meal and following a high-fat, high-calorie meal showed that food overall had no effect on the minimum and overall exposure to bupropion relative to administration in the fasted state, with the geometric mean ratios and 90% CIs for the comparisons of both Cmiₙ and AUCo-12 contained within the 80-125% bioequivalence range. Administration with food resulted in bupropion Cmax falling just outside the bioequivalence range relative to administration in the fasted state, with the geometric mean ratios (90% CIs) of 117% (107.95% - 127.25%), respectively, for the moderate-fat, moderate-calorie condition versus the fasted condition, and 128% (118.72% - 137.95%), respectively, for the high-fat, high-calorie condition versus fasted condition. Prandial state had no effect on bupropion metabolites or on the PAWC, with all geometric mean ratios and 90% CIs falling within the 80-125% range.

In summary, in individuals at a steady state naltrexone/bupropion therapy, administration of two NB 8/90 tablets following a moderate-fat, moderate-calorie meal or following a high-fat, high-calorie meal resulted in substantially higher naltrexone Cmax and AUCo-12 values, and moderately higher bupropion Cmax values, relative to the fasted state.

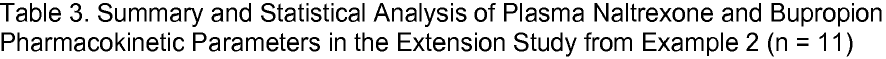

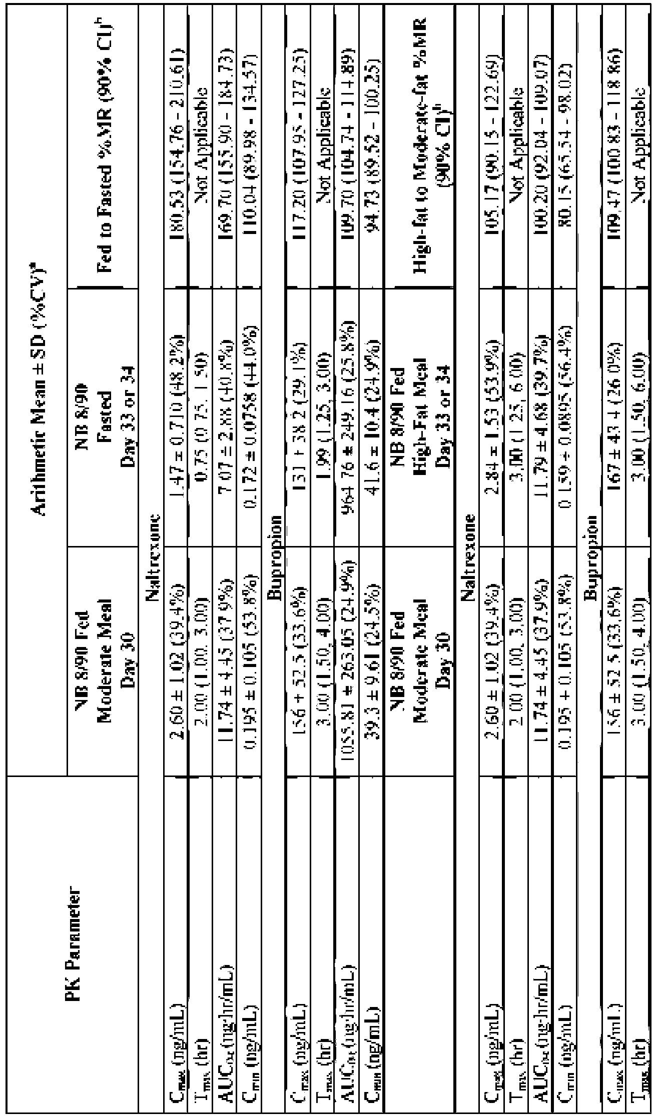

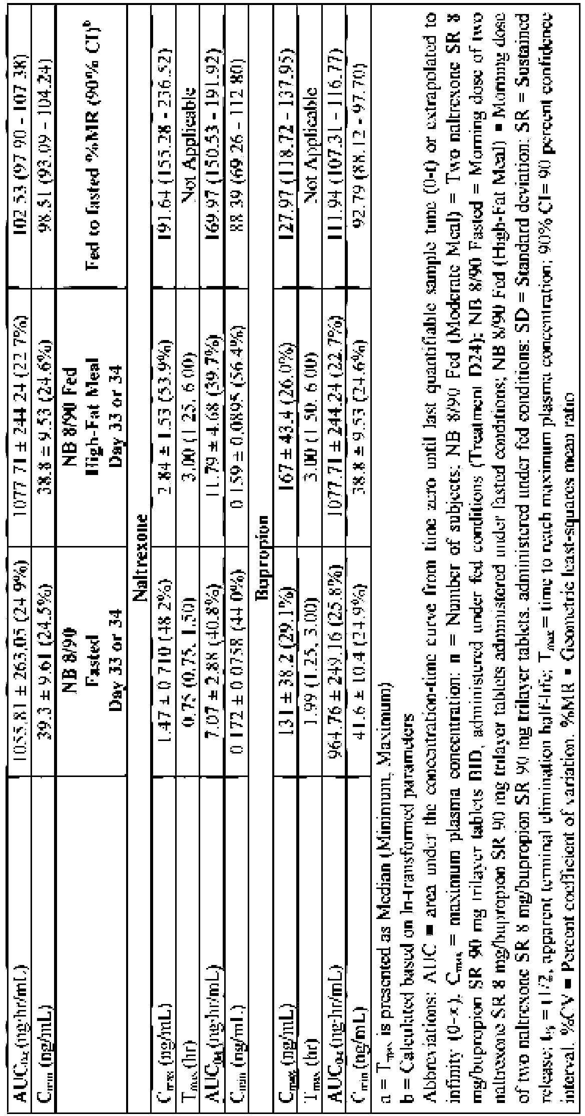

### Example 3 : Naltrexone and Bupropion with a Moderate-Fat, Moderate-Calorie Meal

Two open label, crossover studies were compared to assess the effects of a moderate-fat, moderate-calorie meal to the fasted state on naltrexone and bupropion pharmacokinetics after a single dose. Healthy adult volunteers in a first open label single-dose crossover study (n=20) received two NB 8/90 tablets under a fasted condition. Healthy adult volunteers in a second open label, single-dose crossover study (n=18) received two NB 8/90 tablets administered shortly after a moderate-fat (23%), moderate-calorie (575 kcal) meal (analogous to dietary conditions in Phase 3 trials for weight loss with a combined naltrexone/bupropion therapy).

A summary of food effect comparisons in subjects administered NB 8/90 tablets is also provided in Table 1. Table 4 presents the results of statistical comparisons between the fed (i.e., the second study) and fasted (i.e., the first study) conditions. The moderate-fat, moderate-calorie meal increased naltrexone Cₘₐₓ and AUCo by 114% and 80%), respectively, compared to the fasted condition (i.e., 2.1 -fold and 1.8-fold the value of the fasted condition, respectively). Bupropion pharmacokinetics were not significantly affected by this meal type, with somewhat lower Cₘₐₓ and AUC under fed conditions as compared to fasting conditions. The effect of the moderate-fat, moderate-calorie meal on naltrexone and bupropion pharmacokinetics was thus less marked than the food effect after a high-fat, high-calorie meal under single dose conditions, but remained statistically significant for naltrexone.

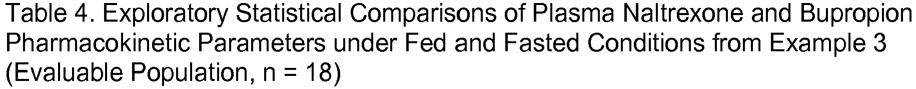

### Example 4: Naltrexone and Bupropion Food Effect Study

A study is conducted to assess the plasma pharmacokinetics of NB tablets under fasted and fed conditions. The treatment groups consist of patients who are instructed to: (1) take a single dose of two NB 8/90 tablets without food; (2) take a single dose of two NB 8/90 tablets with food; or (3) take two NB 8/90 tablets with a food during a steady state of NB 8/90 administration. Blood samples are collected for the determination of plasma

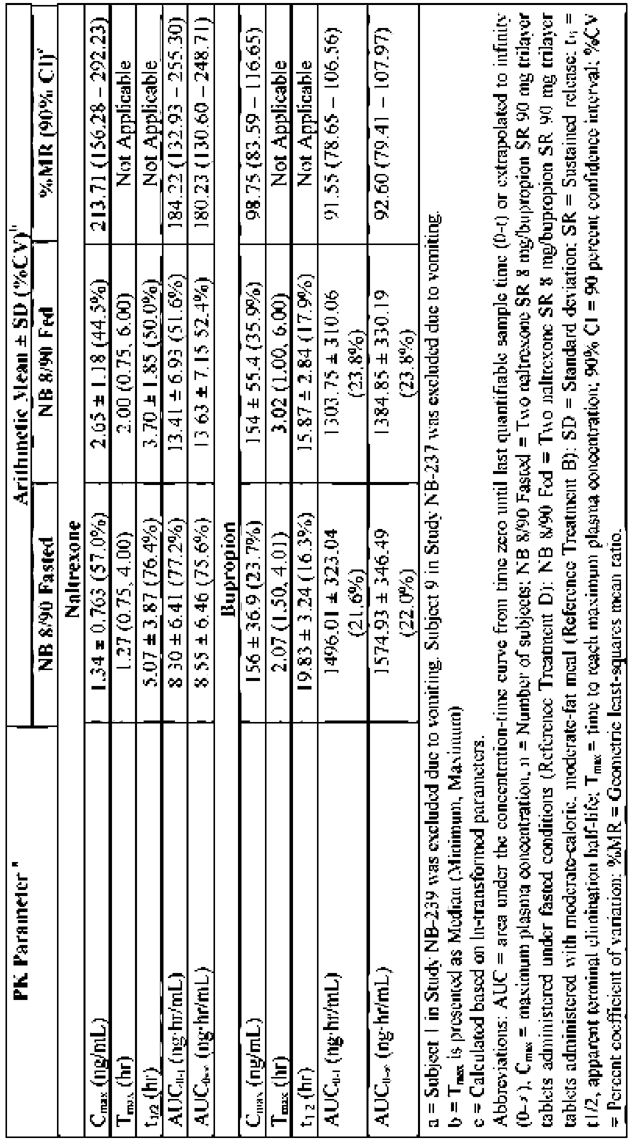

concentrations for naltrexone, bupropion, and their respective metabolites predose (baseline) and at designated time points postdose.

When NB 8/90 is taken with food, the AUC and Cₘₐₓ for naltrexone increase significantly. When NB 8/90 is taken with food during a steady state of NB 8/90 administration, the AUC and Cₘₐₓ for naltrexone increase significantly.

## Claims

1. A pharmaceutical composition for use in the treatment of overweight or obesity comprising a sustained release formulation of naltrexone or a pharmaceutically acceptable salt thereof and a sustained release formulation of bupropion or a pharmaceutically acceptable salt thereof, wherein said composition is administrated in combination with food.

2. The pharmaceutical composition for use according to claim 1, wherein the amount of said bupropion or pharmaceutically acceptable salt thereof is in the range of about 90 mg to about 360 mg per day, and the amount of naltrexone of pharmaceutically acceptable salt thereof is in the range of about 4 mg to about 32 mg per day.

3. The pharmaceutical composition for use according to claim 1, wherein said naltrexone or pharmaceutically acceptable salt thereof comprises a nonsequestered formulation of naltrexone or a pharmaceutically acceptable salt thereof.

4. The pharmaceutical composition for use according to claim 1, wherein said naltrexone or pharmaceutically acceptable salt thereof is administered concurrently with bupropion or a pharmaceutically acceptable salt thereof.

5. The pharmaceutical composition for use according to claim 1, wherein said naltrexone or pharmaceutically acceptable salt thereof is administered prior to or subsequent to bupropion or a pharmaceutically acceptable salt thereof.

6. The pharmaceutical composition for use according to claim 1, wherein said naltrexone or pharmaceutically acceptable salt thereof and said bupropion or pharmaceutically acceptable salt thereof are in a single dosage form.

7. The pharmaceutical composition for use according to any one of the preceding claims, wherein said food comprises a high-fat meal.

8. The pharmaceutical composition for use according to any one of claims 1 to 7, wherein said food comprises a meal selected from the group consisting of a moderate-calorie, moderate-fat meal of about 575 calories and fat accounting for about 23% of the total calorie content; a high-calorie, high-fat meal of about 1000 calories and fat accounting for about 50% of the total calorie content; and a meal that falls within a range defined by said moderate-calorie, moderate-fat meal and said high-calorie, high-fat meal.

9. The pharmaceutical composition for use according to any one of claims 1 to 8, wherein said food comprises a moderate-calorie, moderate-fat meal.

10. The pharmaceutical composition for use according to any one of the preceding claims, wherein the treatment schedule for said naltrexone or pharmaceutically acceptable salt thereof and said bupropion or pharmaceutically acceptable salt thereof is:
about 8 mg of said naltrexone or a pharmaceutically acceptable salt thereof and about 90 mg of said bupropion or a pharmaceuticallyacceptable salt thereof for the first week of treatment;
about 16 mg of said naltrexone or a pharmaceutically acceptablesalt thereof and about 180 mg of said bupropion or a pharmaceuticallyacceptable salt thereof for the second week of treatment;
about 24 mg of said naltrexone or a pharmaceutically acceptable salt thereof and about 270 mg of said bupropion or a pharmaceutically acceptable salt thereof for the third week of treatment; and
about 32 mg of said naltrexone or a pharmaceutically acceptable salt thereof and about 360 mg of said bupropion or a pharmaceutically acceptable salt thereof for the fourth and any subsequent weeks of treatment.

11. The pharmaceutical composition for use according to any one of the preceding claims, wherein said naltrexone or pharmaceutically acceptable salt thereof and said bupropion or pharmaceutically acceptable salt thereof are administered as two 8 mg tablets of sustained-release naltrexone twice daily and two 90 mg tablets of sustained-release bupropion twice daily.

12. The pharmaceutical composition for use according to any one of the preceding claims, wherein said naltrexone or pharmaceutically acceptable salt thereof and said bupropion or pharmaceutically acceptable salt thereof are administered for at least 28 weeks.

13. The pharmaceutical composition for use according to any one of the preceding claims, wherein said naltrexone or pharmaceutically acceptable salt thereof and said bupropion or pharmaceutically acceptable salt thereof are administered for at least 56 weeks.

## Patentansprüche

1. Pharmazeutische Zusammensetzung für die Verwendung in der Behandlung von Übergewicht oder Adipositas, die eine Retardformulierung von Naltrexon oder eines pharmazeutisch akzeptablen Salzes davon sowie eine Retardformulierung von Bupropion oder eines pharmazeutisch akzeptablen Salzes davon aufweist, wobei diese Zusammensetzung zu den Mahlzeiten verabreicht wird.

2. Pharmazeutische Zusammensetzung für die Verwendung nach Anspruch 0, wobei die Menge des Bupropions oder des pharmazeutisch akzeptablen Salzes davon im Bereich von ca. 90 mg bis ca. 360 mg pro Tag und die Menge des Naltrexons oder pharmazeutisch akzeptablen Salzes davon im Bereich von ca. 4 mg bis ca. 32 mg pro Tag liegt.

3. Pharmazeutische Zusammensetzung für die Verwendung nach Anspruch 0, wobei das Naltrexon oder das pharmazeutisch akzeptable Salz davon eine nicht sequestrierte Formulierung von Naltrexon oder einem pharmazeutisch akzeptablen Salz davon enthält.

4. Pharmazeutische Zusammensetzung für die Verwendung nach Anspruch 0, wobei das Naltrexon oder das pharmazeutisch akzeptable Salz davon eine nicht sequestrierte Formulierung von Naltrexon oder einem pharmazeutisch akzeptablen Salz davon enthält.

5. Pharmazeutische Zusammensetzung für die Verwendung nach Anspruch 0, wobei das Naltrexon oder das pharmazeutisch akzeptable Salz davon vor oder nach Bupropion oder einem pharmazeutisch akzeptablen Salz davon verabreicht wird.

6. Pharmazeutische Zusammensetzung für die Verwendung nach Anspruch 0, wobei das Naltrexon oder das pharmazeutisch akzeptable Salz davon und das Bupropion oder das pharmazeutisch akzeptable Salz davon als Einzeldosierungsform bereitgestellt werden.

7. Pharmazeutische Zusammensetzung nach einem der vorstehenden Ansprüche, wobei die Mahlzeit eine fettreiche Mahlzeit umfasst.

8. Pharmazeutische Zusammensetzung nach einem der Ansprüche 0 bis 0, wobei die Mahlzeit eine Mahlzeit aus der Gruppe, bestehend aus einer mittelkalorischen, moderat fettreichen Mahlzeit von ca. 575 Kalorien und einem Fettgehalt von ca. 23 % des Gesamtkaloriengehalts; einer hochkalorischen, sehr fettreichen Mahlzeit von ca. 1000 Kalorien und einem Fettgehalt von ca. 50 % des Gesamtkaloriengehalts; und einer Mahlzeit, die in dem Bereich zwischen der mittelkalorischen, moderat fettreichen und der hochkalorischen, sehr fettreichen Mahlzeit angeordnet ist, ausgewählt wird.

9. Pharmazeutische Zusammensetzung für die Verwendung nach einem der Ansprüche 1 bis 0, wobei die Mahlzeit eine mittelkalorische, moderat fettreiche Mahlzeit umfasst.

10. Pharmazeutische Zusammensetzung für die Verwendung nach einem der vorstehenden Ansprüche, wobei der Behandlungsplan mit dem Naltrexon oder dem pharmazeutisch akzeptablen Salz davon und dem Bupropion oder dem pharmazeutisch akzeptablen Salz davon wie folgt aussieht:
ca. 8 mg des Naltrexons oder eines pharmazeutisch akzeptablen Salzes davon und ca. 90 mg des Bupropions oder eines pharmazeutisch akzeptablen Salzes davon in der ersten Behandlungswoche;
ca. 16 mg des Naltrexons oder eines pharmazeutisch akzeptablen Salzes davon und ca. 180 mg des Bupropions oder eines pharmazeutisch akzeptablen Salzes davon in der zweiten Behandlungswoche;
ca. 24 mg des Naltrexons oder eines pharmazeutisch akzeptablen Salzes davon und ca. 270 mg des Bupropions oder eines pharmazeutisch akzeptablen Salzes davon in der dritten Behandlungswoche; und
ca. 32 mg des Naltrexons oder eines pharmazeutisch akzeptablen Salzes davon und ca. 360 mg des Bupropions oder eines pharmazeutisch akzeptablen Salzes davon in der vierten und in allen anschließenden Behandlungswochen.

11. Pharmazeutische Zusammensetzung für die Verwendung nach einem der vorstehenden Ansprüche, wobei das Naltrexon oder das pharmazeutisch akzeptable Salz davon und das Bupropion oder das pharmazeutisch akzeptable Salz davon in Form von zwei Tabletten von 8 mg Naltrexon als Retardformulierung zweimal täglich und zwei Tabletten von 90 mg Bupropion als Retardformulierung zweimal täglich verabreicht wird.

12. Pharmazeutische Zusammensetzung für die Verwendung nach einem der vorstehenden Ansprüche, wobei das Naltrexon oder das pharmazeutisch akzeptable Salz davon und das Bupropion oder das pharmazeutisch akzeptable Salz davon über einen Zeitraum von mindestens 28 Wochen verabreicht wird.

13. Pharmazeutische Zusammensetzung für die Verwendung nach einem der vorstehenden Ansprüche, wobei das Naltrexon oder das pharmazeutisch akzeptable Salz davon und das Bupropion oder das pharmazeutisch akzeptable Salz davon über einen Zeitraum von mindestens 56 Wochen verabreicht wird.

## Revendications

1. Composition pharmaceutique pour utilisation dans le traitement du surpoids ou de l'obésité comprenant une formulation à libération prolongée de naltrexone ou d'un sel pharmaceutiquement acceptable de cet élément et une formulation à libération prolongée de bupropion ou d'un sel pharmaceutiquement acceptable de cet élément, ladite composition étant administrée en association avec un aliment.

2. Composition pharmaceutique pour utilisation selon la revendication 0, dans laquelle la quantité dudit bupropion ou de sel pharmaceutiquement acceptable de cet élément est comprise entre 90 mg environ et 360 mg environ par jour, et la quantité de naltrexone ou d'un sel pharmaceutiquement acceptable de cet élément est comprise entre 4 mg environ et 32 mg environ par jour.

3. Composition pharmaceutique pour utilisation selon la revendication 0, dans laquelle ladite naltrexone ou ledit sel pharmaceutiquement acceptable de cet élément comprend une formulation non séquestrée de naltrexone ou d'un sel pharmaceutiquement acceptable de cet élément.

4. Composition pharmaceutique pour utilisation selon la revendication 0, dans laquelle ladite naltrexone ou ledit sel pharmaceutiquement acceptable de cet élément est administré en même temps que du bupropion ou un sel pharmaceutiquement acceptable de cet élément.

5. Composition pharmaceutique pour utilisation selon la revendication 0, dans laquelle ladite naltrexone ou ledit sel pharmaceutiquement acceptable de cet élément est administré avant ou après du bupropion ou un sel pharmaceutiquement acceptable de cet élément.

6. Composition pharmaceutique pour utilisation selon la revendication 0, dans laquelle ladite naltrexone ou ledit sel pharmaceutiquement acceptable de cet élément et ledit bupropion ou sel pharmaceutiquement acceptable de cet élément se présentent sous forme de dosage unique.

7. Composition pharmaceutique pour utilisation selon l'une quelconque des revendications précédentes, dans laquelle ledit aliment comprend un repas riche en matières grasses.

8. Composition pharmaceutique pour utilisation selon l'une quelconque des revendications 0 à 0, dans laquelle ledit aliment comprend un repas choisi dans le groupe constitué d'un repas modérément calorique avec apport modéré de matières grasses (575 calories, les matières grasses comptant pour 23 % environ de la teneur totale en calories) ; d'un repas hautement calorique avec apport important de matières grasses (1000 calories, les matières grasses comptant pour 50 % environ de la teneur totale en calories) ; et d'un repas situé dans la plage définie par ledit repas à apport modéré en calories et matières grasses et ledit repas à apport important en calories et matières grasses.

9. Composition pharmaceutique pour utilisation selon l'une quelconque des revendications 1 à 0, dans laquelle ledit aliment comprend un repas à apport modéré en calories et matières grasses.

10. Composition pharmaceutique pour utilisation selon l'une quelconque des revendications précédentes, dans laquelle le programme de traitement à ladite naltrexone ou audit sel pharmaceutiquement acceptable de cet élément et audit bupropion ou sel pharmaceutiquement acceptable de cet élément est le suivant :
8 mg environ de ladite naltrexone ou dudit sel pharmaceutiquement acceptable de cet élément et 90 mg environ dudit bupropion ou sel pharmaceutiquement acceptable de cet élément pendant la première semaine de traitement ;
16 mg environ de ladite naltrexone ou dudit sel pharmaceutiquement acceptable de cet élément et 180 mg environ dudit bupropion ou sel pharmaceutiquement acceptable de cet élément pendant la deuxième semaine de traitement ;
24 mg environ de ladite naltrexone ou dudit sel pharmaceutiquement acceptable de cet élément et 270 mg environ dudit bupropion ou sel pharmaceutiquement acceptable de cet élément pendant la troisième semaine de traitement ;
32 mg environ de ladite naltrexone ou dudit sel pharmaceutiquement acceptable de cet élément et 360 mg environ dudit bupropion ou sel pharmaceutiquement acceptable de cet élément pendant la quatrième semaine de traitement et au-delà.

11. Composition pharmaceutique pour utilisation selon l'une quelconque des revendications précédentes, dans laquelle ladite naltrexone ou ledit sel pharmaceutiquement acceptable de cet élément et ledit bupropion ou sel pharmaceutiquement acceptable de cet élément sont administrés sous forme de deux comprimés de 8 mg à libération prolongée de naltrexone deux fois par jour et de deux comprimés de 90 mg de bupropion à libération prolongée deux fois par jour.

12. Composition pharmaceutique pour utilisation selon l'une quelconque des revendications précédentes, dans laquelle ladite naltrexone ou ledit sel pharmaceutiquement acceptable de cet élément et ledit bupropion ou sel pharmaceutiquement acceptable de cet élément sont administrés pendant au moins 28 semaines.

13. Composition pharmaceutique pour utilisation selon l'une quelconque des revendications précédentes, dans laquelle ladite naltrexone ou ledit sel pharmaceutiquement acceptable de cet élément et ledit bupropion ou sel pharmaceutiquement acceptable de cet élément sont administrés pendant au moins 56 semaines.
